(19) Europäisches Patentamt
European Patent Office
Office européen des brevets

(11) EP 0 548 228 B1

(12) EUROPEAN PATENT SPECIFICATION

(45) Date of publication and mention
of the grant of the patent:
**12.08.1998 Bulletin 1998/33**

(51) Int Cl.[6]: **C12N 9/20**, C12N 15/55,
C11D 3/386, C12N 5/14

(21) Application number: 91916986.2

(22) Date of filing: 13.09.1991

(86) International application number:
PCT/DK91/00271

(87) International publication number:
WO 92/05249 (02.04.1992 Gazette 1992/08)

(54) **LIPASE VARIANTS**

LIPASE-VARIANTEN

VARIANTES LIPASIQUES

(84) Designated Contracting States:
**AT BE CH DE DK ES FR GB GR IT LI LU NL SE**

(30) Priority: 13.09.1990 DK 219490
13.09.1990 DK 219590
13.09.1990 DK 219690

(43) Date of publication of application:
**30.06.1993 Bulletin 1993/26**

(73) Proprietor: **NOVO NORDISK A/S**
**2880 Bagsvaerd (DK)**

(72) Inventors:
• SVENDSEN, Allan
DK-3460 Birkeroed (DK)
• CLAUSEN, Ib, Groth
DK-2920 Charlottenlund (DK)
• PATKAR, Shamkant, Anant
DK-2800 Lyngby (DK)
• GORMSEN, Erik
DK-2830 Virum (DK)

(56) References cited:
EP-A- 305 216        EP-A- 375 102
EP-A- 407 225

• Dialog Information Services, File 154, Medline
85-92, Dialog Accession No. 07295905, DAVIS
RC et al: "Hepatic Lipase: Site-Directed
Mutagenesis of a Serine Residue Important for
Catalytic Activity", J Biol Chem, Apr 15 1990,
265(11) p 6291-5.
• Dialog Information Services, File 154, Medline
85-92, Dialog Accession No. 07295773,
SEMENKOVICH CF et al: "In Vitro Expression
and Site-Specific Mutagenesis of the cloned
human Lipoprotein Lipase gene. Potential
N-Linked Glycosylationsite Asparagine 43 is
Important for both Enzyme Activity and
Secretion" J Biol Chem Apr 5 1990, 265 (10) p
5429-33.
• Dialog Information Services, File 357,
Biotechnology Abstracts, Dialog Accession No.
077832, POULOSE A J: "Alteration of Substrate
Specificity of a lipase by site specific
Mutagenesis - Pseudomonas Enzyme
(Conference Abstract)",
Abstr.Pap.Am.Chem.Soc. (195 Meet BTEC47)
1988.
• Nature, Vol. 343, 1990 LEO BRADY et al: "A
serine protease triad forms the catalytic centre
of a Triacylglycerol Lipase", see page 767 - page
770.
• Nature, Vol. 343, 1990 F.K. WINKLER et al:
"Structure of human pancreatic lipase", see
page 771 - page 774.

SEQUENCE LISTING

(1) GENERAL INFORMATION:

(i) APPLICANT: Novo Nordisk A/S

(ii) TITLE OF INVENTION: Lipase Variants

(iii) NUMBER OF SEQUENCES: 2

(iv) CORRESPONDENCE ADDRESS:

(A) ADDRESSEE: Novo Nordisk A/S
(B) STREET: Novo Alle
(C) CITY: Bagsvaerd
(E) COUNTRY: Denmark
(F) ZIP: 2880

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:

(A) APPLICATION NUMBER:
(B) FILING DATE:
(C) CLASSIFICATION:

(vii) PRIOR APPLICATION DATA:

(A) APPLICATION NUMBER: DK 2196/90
(B) FILING DATE: 13-SEP-1990

(vii) PRIOR APPLICATION DATA:

(A) APPLICATION NUMBER: DK 2194/90
(B) FILING DATE: 13-SEP-1990

(vii) PRIOR APPLICATION DATA:

(A) APPLICATION NUMBER: DK 2195/90
(B) FILING DATE: 13-SEP-1990

(viii) ATTORNEY/AGENT INFORMATION:

(A) NAME: Thalsoe-Madsen, Birgit
(C) REFERENCE/DOCKET NUMBER: 3520.204-WO

(ix) TELECOMMUNICATION INFORMATION:

(A) TELEPHONE: +45 4444 8888
(B) TELEFAX: +45 4449 3256
(C) TELEX: 37304

(2) INFORMATION FOR SEQ ID NO:1:

(i) SEQUENCE CHARACTERISTICS:

(A) LENGTH: 918 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: Humicola lanuginosa

(ix) FEATURE:

(A) NAME/KEY: CDS
(B) LOCATION: 1..873

(xi) SEQUENCE DESCRIPTION: SEQ ID NO:1:

```
ATG AGG AGC TCC CTT GTG CTG TTC TTT GTC TCT GCG TGG ACG GCC TTG   48
Met Arg Ser Ser Leu Val Leu Phe Phe Val Ser Ala Trp Thr Ala Leu
  1               5                  10                  15

GCC AGT CCT ATT CGT CGA GAG GTC TCG CAG GAT CTG TTT AAC CAG TTC   96
Ala Ser Pro Ile Arg Arg Glu Val Ser Gln Asp Leu Phe Asn Gln Phe
                 20                  25                  30

AAT CTC TTT GCA CAG TAT TCT GCA GCC GCA TAC TGC GGA AAA AAC AAT  144
Asn Leu Phe Ala Gln Tyr Ser Ala Ala Ala Tyr Cys Gly Lys Asn Asn
             35                  40                  45

GAT GCC CCA GCT GGT ACA AAC ATT ACG TGC ACG GGA AAT GCC TGC CCC  192
Asp Ala Pro Ala Gly Thr Asn Ile Thr Cys Thr Gly Asn Ala Cys Pro
         50                  55                  60

GAG GTA GAG AAG GCG GAT GCA ACG TTT CTC TAC TCG TTT GAA GAC TCT  240
Glu Val Glu Lys Ala Asp Ala Thr Phe Leu Tyr Ser Phe Glu Asp Ser
65                  70                  75                  80

GGA GTG GGC GAT GTC ACC GGC TTC CTT GCT CTC GAC AAC ACG AAC AAA  288
Gly Val Gly Asp Val Thr Gly Phe Leu Ala Leu Asp Asn Thr Asn Lys
                 85                  90                  95

TTG ATC GTC CTC TCT TTC CGT GGC TCT CGT TCC ATA GAG AAC TGG ATC  336
Leu Ile Val Leu Ser Phe Arg Gly Ser Arg Ser Ile Glu Asn Trp Ile
```

```
                    100                        105                        110
GGG AAT CTT AAC TTC GAC TTG AAA GAA ATA AAT GAC ATT TGC TCC GGC   384
Gly Asn Leu Asn Phe Asp Leu Lys Glu Ile Asn Asp Ile Cys Ser Gly
            115                    120                    125

TGC AGG GGA CAT GAC GGC TTC ACT TCG TCC TGG AGG TCT GTA GCC GAT   432
Cys Arg Gly His Asp Gly Phe Thr Ser Ser Trp Arg Ser Val Ala Asp
        130                    135                    140

ACG TTA AGG CAG AAG GTG GAG GAT GCT GTG AGG GAG CAT CCC GAC TAT   480
Thr Leu Arg Gln Lys Val Glu Asp Ala Val Arg Glu His Pro Asp Tyr
145                    150                    155                    160

CGC GTG GTG TTT ACC GGA CAT AGC TTG GGT GGT GCA TTG GCA ACT GTT   528
Arg Val Val Phe Thr Gly His Ser Leu Gly Gly Ala Leu Ala Thr Val
                165                    170                    175

GCC GGA GCA GAC CTG CGT GGA AAT GGG TAT GAT ATC GAC GTG TTT TCA   576
Ala Gly Ala Asp Leu Arg Gly Asn Gly Tyr Asp Ile Asp Val Phe Ser
            180                    185                    190

TAT GGC GCC CCC CGA GTC GGA AAC AGG GCT TTT GCA GAA TTC CTG ACC   624
Tyr Gly Ala Pro Arg Val Gly Asn Arg Ala Phe Ala Glu Phe Leu Thr
            195                    200                    205

GTA CAG ACC GGC GGA ACA CTC TAC CGC ATT ACC CAC ACC AAT GAT ATT   672
Val Gln Thr Gly Gly Thr Leu Tyr Arg Ile Thr His Thr Asn Asp Ile
        210                    215                    220

GTC CCT AGA CTC CCG CCG CGC GAA TTC GGT TAC AGC CAT TCT AGC CCA   720
Val Pro Arg Leu Pro Pro Arg Glu Phe Gly Tyr Ser His Ser Ser Pro
225                    230                    235                    240

GAG TAC TGG ATC AAA TCT GGA ACC CTT GTC CCC GTC ACC CGA AAC GAT   768
Glu Tyr Trp Ile Lys Ser Gly Thr Leu Val Pro Val Thr Arg Asn Asp
                245                    250                    255

ATC GTG AAG ATA GAA GGC ATC GAT GCC ACC GGC GGC AAT AAC CAG CCT   816
Ile Val Lys Ile Glu Gly Ile Asp Ala Thr Gly Gly Asn Asn Gln Pro
            260                    265                    270

AAC ATT CCG GAT ATC CCT GCG CAC CTA TGG TAC TTC GGG TTA ATT GGG   864
Asn Ile Pro Asp Ile Pro Ala His Leu Trp Tyr Phe Gly Leu Ile Gly
            275                    280                    285

ACA TGT CTT TAGTGGCCGG CGCGGCTGGG TCCGACTCTA GCGAGCTCGA GATCT   918
Thr Cys Leu
        290
```

(2) INFORMATION FOR SEQ ID NO:2:

    (i) SEQUENCE CHARACTERISTICS:

        (A) LENGTH: 291 amino acids
        (B) TYPE: amino acid
        (D) TOPOLOGY: linear

    (ii) MOLECULE TYPE: protein

(xi) SEQUENCE DESCRIPTION: SEQ ID NO:2:

```
Met Arg Ser Ser Leu Val Leu Phe Phe Val Ser Ala Trp Thr Ala Leu
 1               5                  10                  15

Ala Ser Pro Ile Arg Arg Glu Val Ser Gln Asp Leu Phe Asn Gln Phe
             20                  25                  30

Asn Leu Phe Ala Gln Tyr Ser Ala Ala Ala Tyr Cys Gly Lys Asn Asn
         35                  40                  45

Asp Ala Pro Ala Gly Thr Asn Ile Thr Cys Thr Gly Asn Ala Cys Pro
     50                  55                  60

Glu Val Glu Lys Ala Asp Ala Thr Phe Leu Tyr Ser Phe Glu Asp Ser
 65                  70                  75                  80

Gly Val Gly Asp Val Thr Gly Phe Leu Ala Leu Asp Asn Thr Asn Lys
                 85                  90                  95

Leu Ile Val Leu Ser Phe Arg Gly Ser Arg Ser Ile Glu Asn Trp Ile
             100                 105                 110

Gly Asn Leu Asn Phe Asp Leu Lys Glu Ile Asn Asp Ile Cys Ser Gly
         115                 120                 125

Cys Arg Gly His Asp Gly Phe Thr Ser Ser Trp Arg Ser Val Ala Asp
     130                 135                 140

Thr Leu Arg Gln Lys Val Glu Asp Ala Val Arg Glu His Pro Asp Tyr
145                 150                 155                 160

Arg Val Val Phe Thr Gly His Ser Leu Gly Gly Ala Leu Ala Thr Val
             165                 170                 175

Ala Gly Ala Asp Leu Arg Gly Asn Gly Tyr Asp Ile Asp Val Phe Ser
```

```
                    180                         185                          190

        Tyr Gly Ala Pro Arg Val Gly Asn Arg Ala Phe Ala Glu Phe Leu Thr
                195                 200                 205

        Val Gln Thr Gly Gly Thr Leu Tyr Arg Ile Thr His Thr Asn Asp Ile
                210                 215                 220

        Val Pro Arg Leu Pro Pro Arg Glu Phe Gly Tyr Ser His Ser Ser Pro
        225                 230                 235                 240

        Glu Tyr Trp Ile Lys Ser Gly Thr Leu Val Pro Val Thr Arg Asn Asp
                        245                 250                 255

        Ile Val Lys Ile Glu Gly Ile Asp Ala Thr Gly Gly Asn Asn Gln Pro
                        260                 265                 270

        Asn Ile Pro Asp Ile Pro Ala His Leu Trp Tyr Phe Gly Leu Ile Gly
                275                 280                 285

        Thr Cys Leu
                290
```

**Description**

FIELD OF INVENTION

The present invention relates to novel lipase enzyme variants with improved properties, DNA constructs coding for the expression of said variants, host cells capable of expressing the variants from the DNA constructs, as well as a method of producing the variants by cultivating said host cells.

BACKGROUND OF THE INVENTION

The advent and development of recombinant DNA techniques has had a profound influence on the field of protein chemistry. It has been envisaged that these techniques will make it possible to design peptides and proteins, such as enzymes, in accordance with specific criteria, thus permitting the production of compounds with desired properties.

Due to the availability of such techniques, it has become possible to construct enzymes with desired amino acid sequences, and a fair amount of research has been devoted to this object.

The primary structure of a number of lipases has been determined and described in the litterature (Boel et al., *Lipids* **23**, 701-706 (1988), de Caro et al., *Biochim. Biophys. Acta* **671,** 129-138 (1981), Winkler et al., *Nature* **343**, 771-774 (1990)). Furthermore also the tertiary structure of a more limited number of lipases has been elucidated (Winkler et al., *Nature* **343,** 771-774 (1990), Brady et al., *Nature* **343,** 767-770 (1990) J.D. Schrag et al., Nature 351, 1991, pp. 761-764). From these investigations it appears that lipases seem to have certain structural features in common, but that,on the other hand, major structural variations also exist among the lipases.

SUMMARY OF THE INVENTION

Further investigations have now shown that improved properties of lipases may be obtained by one or more specific mutations in the DNA sequence expressing a specific lipase in order to obtain lipase variants exhibiting such improved properties.

Consequently, in one aspect, the present invention relates to a lipase variant of a parent lipase comprising a trypsin-like catalytic triad including an active serine located in a predominantly hydrophobic, elongated binding pocket of the lipase molecule, wherein the electrostatic charge and/or hydrophobicity of the lipid contact zone of the parent lipase is changed by deleting or substituting one or more negatively charged amino acid residues by neutral or positively charged amino acid residue(s), and/or by substituting one or more neutral amino acid residues by positively charged amino acid residue(s), and/or by deleting or substituting one or more hydrophilic amino acid residues by hydrophobic amino acid residue(s). For the sake of convenience, this lipase variant is termed lipase variant I in the following.

In the present context, the term "trypsin-like" is intended to indicate that the parent lipase comprises a catalytic triad at the active site corresponding to that of trypsin, i.e. the amino acids Ser, His and one of Asp, Glu, Asn or Gln. Some lipases may also comprise a surface loop structure which covers the active serine when the lipase is in inactive form (an example of such a lipase is described by Brady et al., Nature 343, 1990, pp. 767-770). When the lipase is activated, the loop structure is shifted to expose the active site residues, creating a surface with increased surface hydrophobicity which interacts with the lipid substrate at or during hydrolysis. For the present purpose, this surface is termed the "lipid contact zone", intended to include amino acid residues located within or forming part of this surface. These residues may participate in lipase interaction with the substrate at or during hydrolysis where the lipase hydrolyses triglycerides from the lipid phase when activated by contact with the lipid surface. During hydrolysis of the triglycerides, fatty acids and mono- and di-glycerides are formed in varying amounts. One reason for changing the electrostatic charge and/or hydrophobicity of the lipid contact zone by mutating the lipase in that zone is that the fatty acids formed during hydrolysis may remain in the lipid phase, thus forming a negatively charged surface. When the lipase is used for washing purposes, negatively charged detergents may form negative charges on the lipid surface. Thus, by preparing lipase variants which are less negatively charged and/or more hydrophobic, it may be possible to obtain lipases with different specificities and/or improved properties.

The present invention also relates to a DNA construct comprising a DNA sequence encoding a lipase variant as indicated above, a recombinant expression vector carrying said DNA construct, a cell transformed with the DNA construct or the expression vector, as well as a method of producing a lipase variant of the invention by culturing or growing said cell under conditions conducive to the production of the lipase variant, after which the lipase variant is recovered from the culture.

The invention further relates to a detergent additive comprising a lipase variant of the invention, optionally in the form of a non-dusting granulate, stabilised liquid or protected enzyme, as well as to a detergent composition comprising lipase variant of the invention.

DETAILED DISCLOSURE OF THE INVENTION

In the present description and claims, the following abbreviations are used:

Amino acids:

| A | = Ala | = Alanine |
|---|-------|-----------|
| V | = Val | = Valine |
| L | = Leu | = Leucine |
| I | = Ile | = Isoleucine |
| P | = Pro | = Proline |
| F | = Phe | = Phenylalanine |
| W | = Trp | = Tryptophan |
| M | = Met | = Methionine |
| G | = Gly | = Glycine |
| S | = Ser | = Serine |
| T | = Thr | = Threonine |
| C | = Cys | = Cysteine |
| Y | = Tyr | = Tyrosine |
| N | = Asn | = Asparagine |
| Q | = Gln | = Glutamine |
| D | = Asp | = Aspartic Acid |
| E | = Glu | = Glutamic Acid |
| K | = Lys | = Lysine |
| R | = Arg | = Arginine |
| H | = His | = Histidine |

In describing lipase variants according to the invention, the following nomenclature is used for ease of reference:
Original amino acid(s):position(s):substituted amino acid(s)
According to this nomenclature, for instance the substitution of glutamic acid for glycine in position 195 is shown as:

Gly 195 Glu    or    G195E

a deletion of glycine in the same position is shown as:

Gly 195 *    or    G195*

and insertion of an additional amino acid residue such as lysine is shown as:

Gly 195 GlyLys or    G195GK

Where a specific lipase contains a "deletion" in comparison with other lipases and an insertion is made in such a position this is indicated as:

* 36 Asp    or    *36D

for insertion of an aspartic acid in position 36
Multiple mutations are separated by pluses, i.e.:

```
Arg 170 Tyr + Gly 195 Glu          or    R170Y+G195E
```

representing mutations in positions 170 and 195 substituting tyrosine and glutamic acid for arginine and glycine, respectively.

According to the invention, lipase variant I is preferably one in which one or more glutamic acid or aspartic acid residues of the lipid contact zone of the lipase are substituted by glutamine, asparagine, alanine, leucine, valine, serine, threonine, lysine, or arginine.

Although the parent lipase may be derived from a variety of sources such as mammalian lipases, e.g. pancreatic, gastric, hepatic or lipoprotein lipases, it is generally preferred that it is a microbial lipase. As such, the parent lipase may be selected from yeast, e.g. Candida, lipases, bacterial, e.g. Pseudomonas, lipases or fungal, e.g. Humicola or Rhizomucor lipases. It is particularly preferred to select the parent lipase from a group of structurally homologous lipases.

In a preferred embodiment of lipase variant I of the invention, the parent lipase is a Rhizomucor miehei lipase, in particular the lipase described in EP 305 216. In this embodiment, one or more negatively charged amino acid residues may be substituted by one or more positively charged or neutral amino acid residues as follows

D91N,K,R,A,V,L,S,T;
D256N,K,R,A,V,L,S,T;
D226N,K,R,A,V,L,S,T;
D61N,K,R,A,V,L,S,T;
D113N,K,R,A,V,L,S,T;
E201Q,K,R,A,V,L,S,T;
D243N,K,R,A,V,L,S,T.

In another preferred embodiment of lipase variant I of the invention, the parent lipase is a Humicola lanuginosa lipase, in particular the lipase produced by H. lanuginosa strain DSM 4106 (cf. EP 258 068). In this embodiment, one or more negatively charged amino acid residues may be substituted by one or more neutral or positively charged amino acid residues as follows:

E87Q,K,R,A,N,T,S,L,V;
D254N,K,R,A,Q,T,S,L,V;
D242N,K,R,A,Q,T,S,L,V;
E210Q,K,R,A,N,T,S,L,V;
E56Q,K,R,A,N,T,S,L,V;
D96N,K,R,A,Q,T,S,L,V;
DIIIN,K,R,A,Q,T,S,L,V;
D62A,Q,N,T,S,K,R,L,V;
E219A,Q,N,T,S,K,R,L,V;
E234A,Q,N,T,S,K,R,L,V;
E57A,Q,N,T,S,K,R,L,V
E99A,Q,N,T,S,K,R,L,V;
D27A,Q,N,T,S,K,R,L,V; or
E239A,Q,N,T,S,K,R,L,V.

Particularly preferred substitutions according to the invention are

E87Q + D254N + D242N + E210Q;
E87Q + D254N + E210Q;
D96N + E87Q + D254N:
R209A + E210A.

Alternatively, one or more neutral amino acid residues may be substituted by one or more positively charged amino acid residues as follows:

T267K,R;
S85K,R;
T226K,R;

N88K,R;
N92K,R;
I255K,R;
I202K,R
L206K,R;
L259K,R;
V203K,R; or
L227K,R

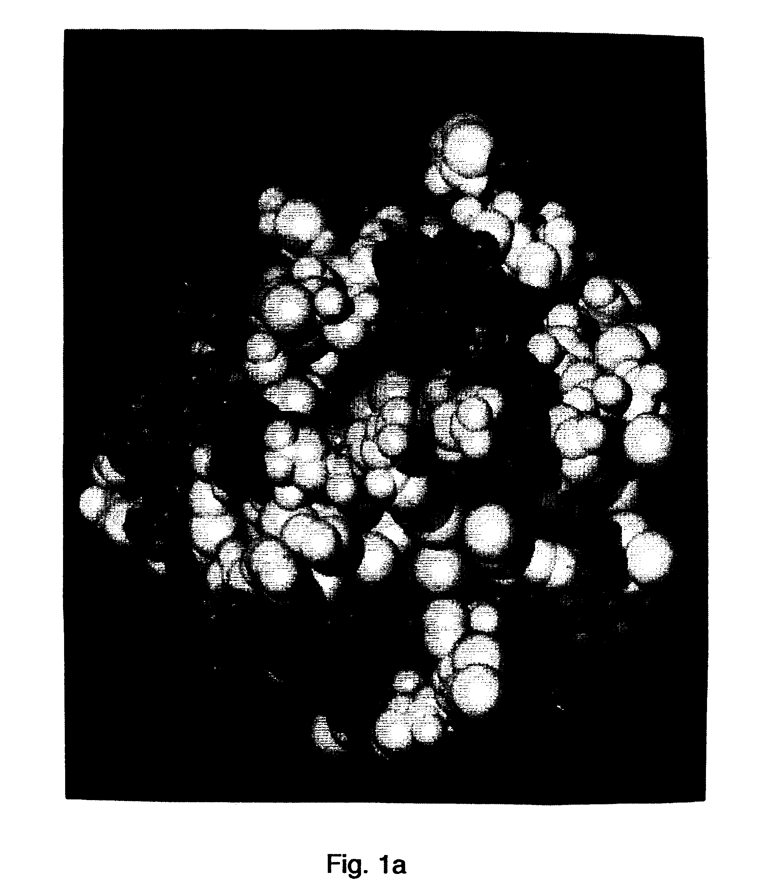

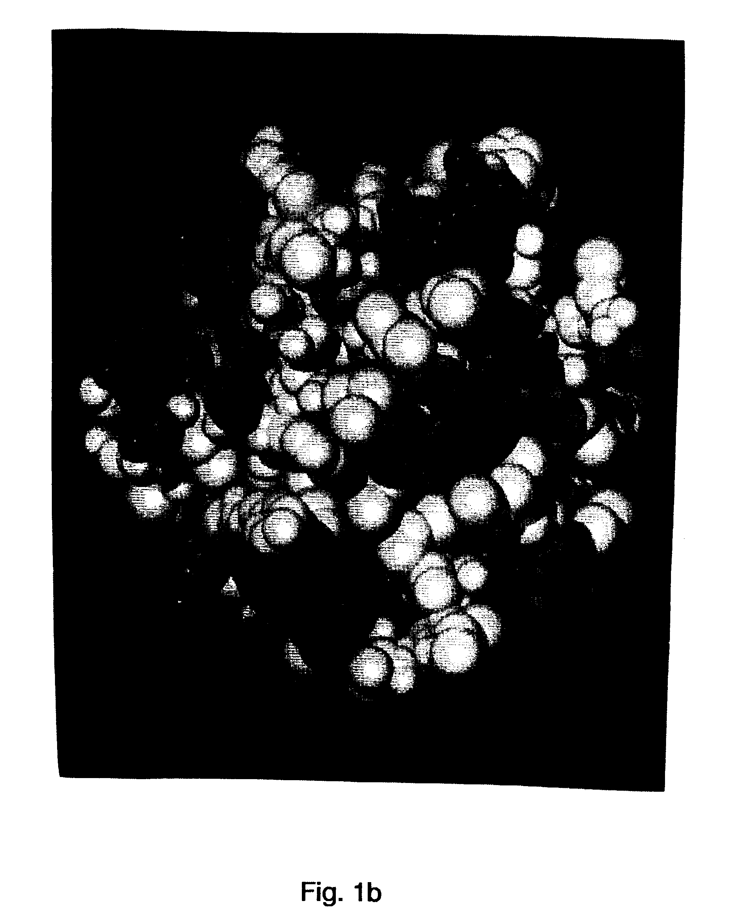

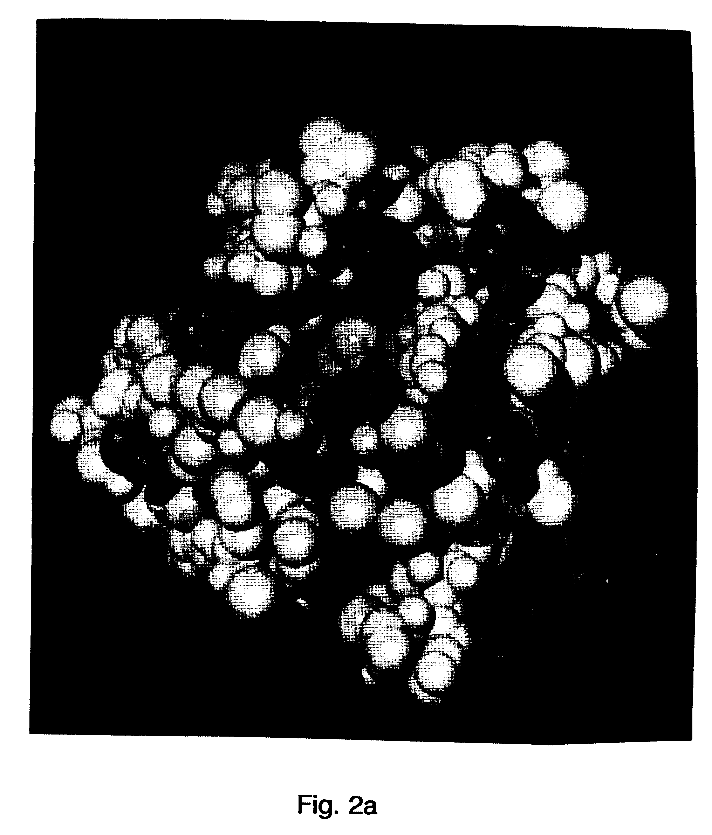

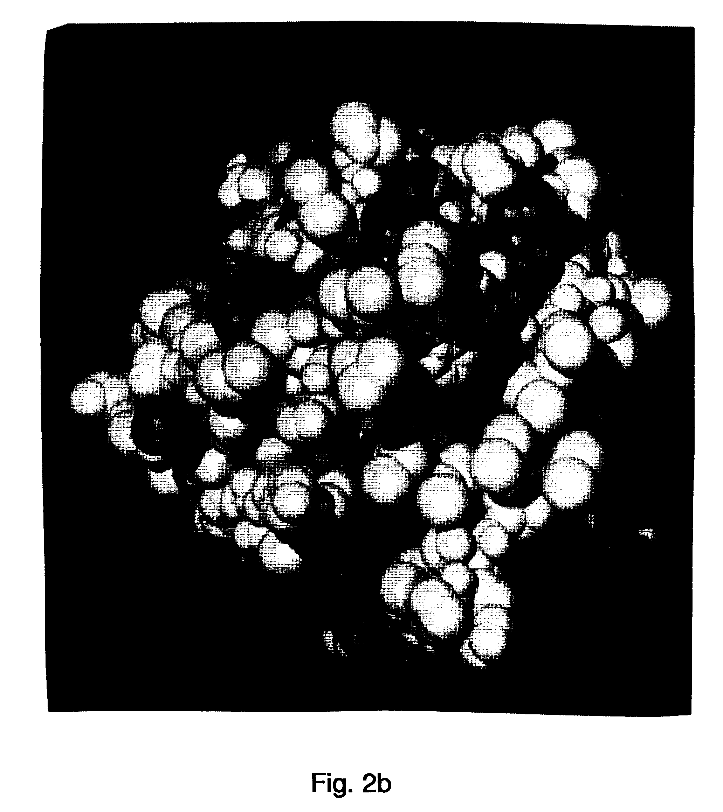

It should be noted that the Humicola lanuginosa lipase and the Rhizomucor miehei lipase belong to the same group of lipases. This implies that the overall three-dimensional structure of the two lipases is very similar and has been shown by X-ray crystallography to be highly homologous (a computer model of the H. lanuginosa and the Rh. miehei lipase is shown in Figs. 1A and B and 2A and B, respectively, from which the similarities between the lipid contact zones of the two lipases are clearly apparent). It is therefore probable that modifications of the type indicated for either lipase will also be functional for the other lipase.

It should be noted that, according to the invention, any one of the modifications of the amino acid sequence indicated above for the lipase variant I may be combined with any one of the other modifications mentioned above or any one of the modifications for variants II and III described in WO 92/05249.

Methods of preparing lipase variants of the invention

Several methods for introducing mutations into genes are known in the art. After a brief discussion of cloning lipase-encoding

DNA sequences, methods for generating mutations at specific sites within the lipase-encoding sequence will be discussed.

Cloning a DNA sequence encoding a lipase

The DNA sequence encoding a parent lipase may be isolated from any cell or microorganism producing the lipase in question by various methods, well known in the art. First a genomic DNA and/or cDNA library should be constructed using chromosomal DNA or messenger RNA from the organism that produces the lipase to be studied. Then, if the amino acid sequence of the lipase is known, homologous, labelled oligonucleotide probes may be synthesized and used to identify lipase-encoding clones from a genomic library of bacterial DNA, or from a fungal cDNA library. Alternatively, a labelled oligonucleotide probe containing sequences homologous to lipase from another strain of bacteria or fungus could be used as a probe to identify lipase-encoding clones, using hybridization and washing conditions of lower stringency.

Yet another method for identifying lipase-producing clones would involve inserting fragments of genomic DNA into an expression vector, such as a plasmid, transforming lipase-negative bacteria with the resulting genomic DNA library, and then plating the transformed bacteria onto agar containing a substrate for lipase. Those bacteria containing lipase-bearing plasmid will produce colonies surrounded by a halo of clear agar, due to digestion of the substrate by secreted lipase.

Alternatively, the DNA sequence encoding the enzyme may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., The EMBO J. 3, 1984, pp. 801-805. According to the phosphoamidite method, oligonucleotides are synthesized, e.g. in an automatic DNA synthesizer, purified, annealed, ligated and cloned in appropriate vectors.

Finally, the DNA sequence may be of mixed genomic and synthetic, mixed synthetic and cDNA or mixed genomic and cDNA origin prepared by ligating fragments of synthetic, genomic or cDNA origin (as appropriate), the fragments corresponding to various parts of the entire DNA sequence, in accordance with standard techniques. The DNA sequence may also be prepared by polymerase chain reaction (PCR) using specific primers, for instance as described in US 4,683,202 or R.K. Saiki et al., Science 239, 1988, pp. 487-491.

Site-directed mutagenesis of the lipase-encoding sequence

Once a lipase-encoding DNA sequence has been isolated, and desirable sites for mutation identified, mutations may be introduced using synthetic oligonucleotides. These oligonucleotides contain nucleotide sequences flanking the desired mutation sites; mutant nucleotides are inserted during oligonucleotide synthesis. In a specific method, a single-stranded gap of DNA, bridging the lipase-encoding sequence, is created in a vector carrying the lipase gene. Then the synthetic nucleotide, bearing the desired mutation, is annealed to a homologous portion of the single-stranded DNA. The remaining gap is then filled in with DNA polymerase I (Klenow fragment) and the construct is ligated using T4 ligase. A specific example of this method is described in Morinaga et al., (1984, Biotechnology 2:646-639). U.S. Patent number 4,760,025, by Estell et al., issued July 26, 1988, discloses the introduction of oligonucleotides encoding multiple mutations by performing minor alterations of the cassette, however, an even greater variety of mutations can be introduced at any one time by the Morinaga method, because a multitude of oligonucleotides, of various lengths, can be introduced.

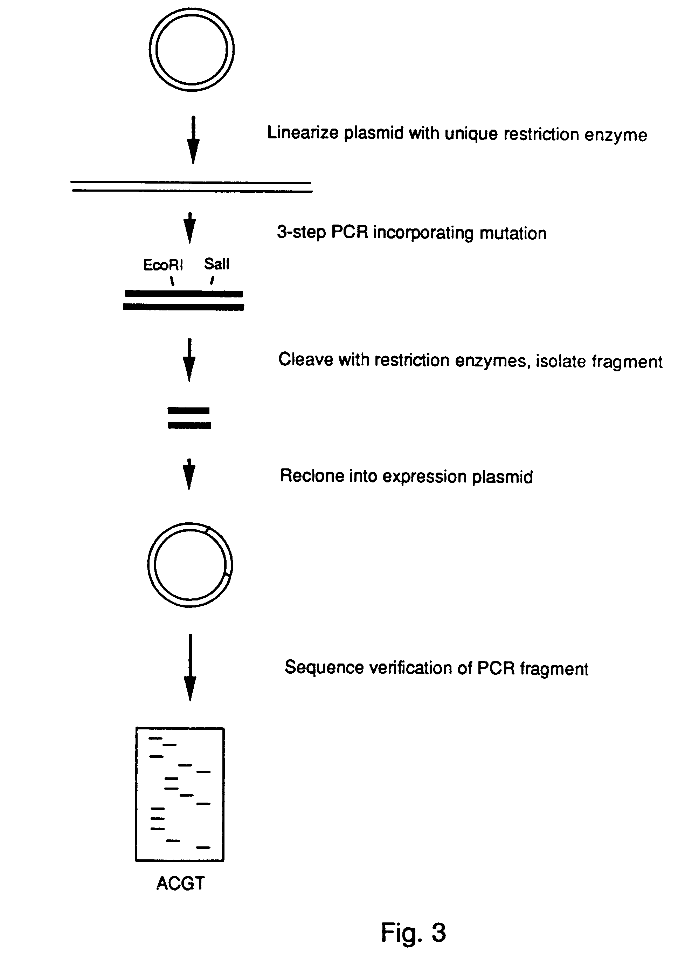

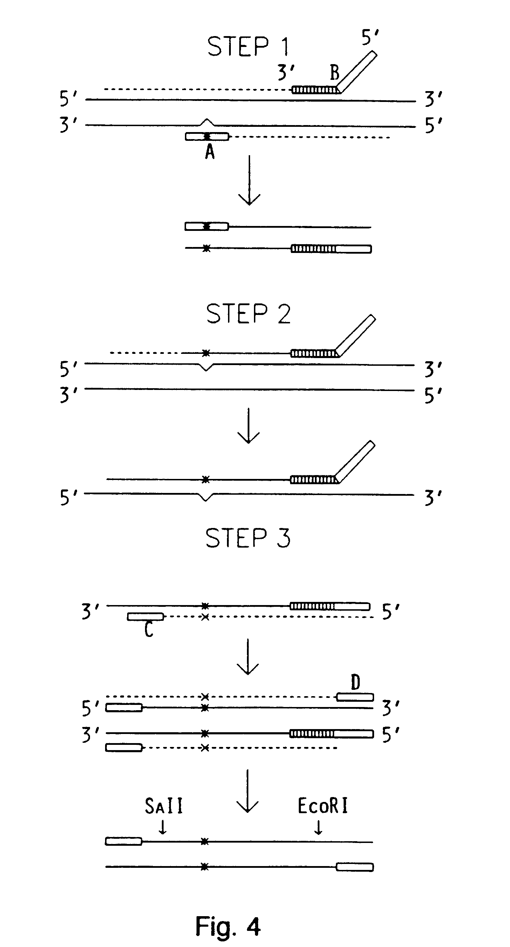

Another method of introducing mutations into lipase-encoding sequences is described in Nelson and Long, Analytical Biochemistry 180, 1989, pp. 147-151. It involves the 3-step generation of a PCR fragment containing the desired mutation introduced by using a chemically synthesized DNA strand as one of the primers in the PCR reactions. From the PCR-generated fragment, a DNA fragment carrying the mutation may be isolated by cleavage with restriction endonucleases and reinserted into an expression plasmid (see also Figs. 3 and 4 where this method is further outlined).

Expression of lipase variants

According to the invention, a mutated lipase-coding sequence produced by methods described above, or any alternative methods known in the art, can be expressed, in enzyme form, using an expression vector which typically includes control sequences encoding a promoter, operator, ribosome binding site, translation initiation signal, and, optionally, a repressor gene or various activator genes. To permit the secretion of the expressed protein, nucleotides encoding a "signal sequence" may be inserted prior to the lipase-coding sequence. For expression under the direction of control sequences, a target gene to be treated according to the invention is operably linked to the control sequences in the proper reading frame. Promoter sequences that can be incorporated into plasmid vectors, and which can support the transcription of the mutant lipase gene, include but are not limited to the prokaryotic β-lactamase promoter (Villa-Kamaroff, et al., 1978, Proc. Natl. Acad. Sci. U.S.A. 75:3727-3731) and the tac promoter (DeBoer, et al., 1983, Proc. Natl. Acad. Sci. U.S.A. 80:21-25). Further references can also be found in "Useful proteins from recombinant bacteria" in Scientific American, 1980, 242:74-94.

According to one embodiment B. subtilis is transformed by an expression vector carrying the mutated DNA. If expression is to take place in a secreting microorganism such as B. subtilis a signal sequence may follow the translation initiation signal and precede the DNA sequence of interest. The signal sequence acts to transport the expression product to the cell wall where it is cleaved from the product upon secretion. The term "control sequences" as defined above is intended to include a signal sequence, when is present.

In a currently preferred method of producing lipase variants of the invention, a filamentous fungus is used as the host organism. The filamentous fungus host organism may conveniently be one which has previously been used as a host for producing recombinant proteins, e.g. a strain of Aspergillus sp., such as A. niger, A. nidulans or A. oryzae. The use of A. oryzae in the production of recombinant proteins is extensively described in, e.g. EP 238 023.

For expression of lipase variants in Aspergillus, the DNA sequence coding for the lipase variant is preceded by a promoter. The promoter may be any DNA sequence exhibiting a strong transcriptional activity in Aspergillus and may be derived from a gene encoding an extracellular or intracellular protein such as an amylase, a glucoamylase, a protease, a lipase, a cellulase or a glycolytic enzyme.

Examples of suitable promoters are those derived from the gene encoding A. oryzae TAKA amylase, Rhizomucor miehei aspartic proteinase, A. niger neutral α-amylase, A. niger acid stable α-amylase, A. niger glucoamylase, Rhizomucor miehei lipase, A. oryzae alkaline protease or A. oryzae triose phosphate isomerase.

In particular when the host organism is A. oryzae, a preferred promoter for use in the process of the present invention is the A. oryzae TAKA amylase promoter as it exhibits a strong transcriptional activity in A. oryzae. The sequence of the TAKA amylase promoter appears from EP 238 023.

Termination and polyadenylation sequences may suitably be derived from the same sources as the promoter.

The techniques used to transform a fungal host cell may suitably be as described in EP 238 023.

To ensure secretion of the lipase variant from the host cell, the DNA sequence encoding the lipase variant may be preceded by a signal sequence which may be a naturally occurring signal sequence or a functional part thereof or a synthetic sequence providing secretion of the protein from the cell. In particular, the signal sequence may be derived from a gene encoding an Aspergillus sp. amylase or glucoamylase, a gene encoding a Rhizomucor miehei lipase or protease, or a gene encoding a Humicola cellulase, xylanase or lipase. The signal sequence is preferably derived from the gene encoding A. oryzae TAKA amylase, A. niger neutral α-amylase, A. niger acid-stable α-amylase or A. niger glucoamylase.

The medium used to culture the transformed host cells may be any conventional medium suitable for growing Aspergillus cells. The transformants are usually stable and may be cultured in the absence of selection pressure. However, if the transformants are found to be unstable, a selection marker introduced into the cells may be used for selection.

The mature lipase protein secreted from the host cells may conveniently be recovered from the culture medium by well-known procedures including separating the cells from the medium by centrifugation or filtration, and precipitating proteinaceous components of the medium by means of a salt such as ammonium sulphate, followed by chromatographic procedures such as ion exchange chromatography, affinity chromatography, or the like.

The present invention also relates to a detergent additive comprising a lipase variant according to the invention, preferably in the form of a non-dusting granulate, stabilized liquid or protected enzyme. Non-dusting granulates may be produced e.g. according to US 4,106,991 and 4,661,452 (both to Novo Industri A/S) and may optionally be coated by methods known in the art. Liquid enzyme preparations may, for instance, be stabilized by adding a polyol such as propylene glycol, a sugar or sugar alcohol, lactic acid or boric acid according to established methods. Other enzyme stabilizers are well known in the art. Protected enzymes may be prepared according to the method disclosed in EP 238 216.

The detergent additive may suitably contain 0.02-200 mg of enzyme protein per gram of the additive. It will be understood that the detergent additive may further include one or more other enzymes, such as a protease, cellulase, peroxidase or amylase, conventionally included in detergent additives.

In a still further aspect, the invention relates to a detergent composition comprising a lipase variant of the invention. Detergent compositions of the invention additionally comprise surfactants which may be of the anionic, non-ionic, cationic, amphoteric, or zwitterionic type as well as mixtures of these surfactant classes. Typical examples of suitable surfactants are linear alkyl benzene sulfonates (LAS), alpha olefin sulfonates (AOS), alcohol ethoxy sulfates (AEOS), alcohol ethoxylates (AEO), alkyl sulphates (AS), alkyl polyglycosides (APG) and alkali metal salts of natural fatty acids.

Detergent compositions of the invention may contain other detergent ingredients known in the art as e.g. builders, bleaching agents, bleach activators, anti-corrosion agents, sequestering agents, anti soil-redeposition agents, perfumes, enzyme stabilizers, etc.

The detergent composition of the invention may be formulated in any convenient form, e.g. as a powder or liquid. The enzyme may be stabilized in a liquid detergent by inclusion of enzyme stabilizers as indicated above. Usually, the pH of a solution of the detergent composition of the invention will be 7-12 and in some instances 7.0-10.5. Other detergent enzymes such as proteases, cellulases, peroxidases or amylases may be included the detergent compositions of the invention, either separately or in a combined additive as described above.

BRIEF DESCRIPTION OF THE DRAWINGS

The present invention is described in the following with reference to the appended drawings, in which

Fig. 1A and B are computer models showing the three-dimensional structure of the lipid contact zone of the H. lanuginosa lipase when the lipase is in inactive (A) and active (B) form, respectively. "White" residues represent hydrophobic amino acids (Ala, Val, Leu, Ile, Pro, Phe, Trp, Gly and Met), "yellow" residues represent hydrophilic amino acids (Thr, Ser, Gln, Asn, Tyr and Cys), "blue" residues represent positively charged amino acids (Lys, Arg and His), and "red" residues represent negatively charged amino acids (Glu and Asp);

Fig. 2A and 2B are computer models showing the three-dimensional structure of the lipid contact zone of the Rh. miehei lipase when the lipase is in inactive (A) and active (B) form, respectively. "White" residues represent hydrophobic amino acids (Ala, Val, Leu, Ile, Pro, Phe, Trp, Gly and Met), "yellow" residues represent hydrophilic amino acids (Thr, Ser, Gln, Asn, Tyr and Cys), "blue" residues represent positively charged amino acids (Lys, Arg and His), and "red" residues represent negatively charged amino acids (Glu and Asp);

Fig. 3 is a schematic representation of the preparation of plasmids encoding lipase variants by polymerase chain reaction (PCR);

Fig. 4 is a schematic representation of the three-step mutagenesis by PCR;

Fig. 5 shows a restriction map of plasmid pAO1;

Fig. 6 shows a restriction map of plasmid pAHL; and

Fig. 7 shows a restriction map of plasmid pARML.

The present invention is further illustrated in the following examples which are not in any way intended to limit the scope of the invention as claimed.

GENERAL METHODS

**Expression of Humicola lanuginosa lipase and Rhizomucor miehei lipase in Aspergillus oryzae:**

Cloning of Humicola lanuginosa lipase and Rhizomucor miehei lipase is described in EP 305,216 and EP 238 023, respectively. These patent applications also describe expression and characterization of the two lipases in Aspergillus oryzae. The two expression plasmids used are termed p960 (carrying the H. lanuginosa lipase gene) and p787 (carrying the R. miehei lipase gene).

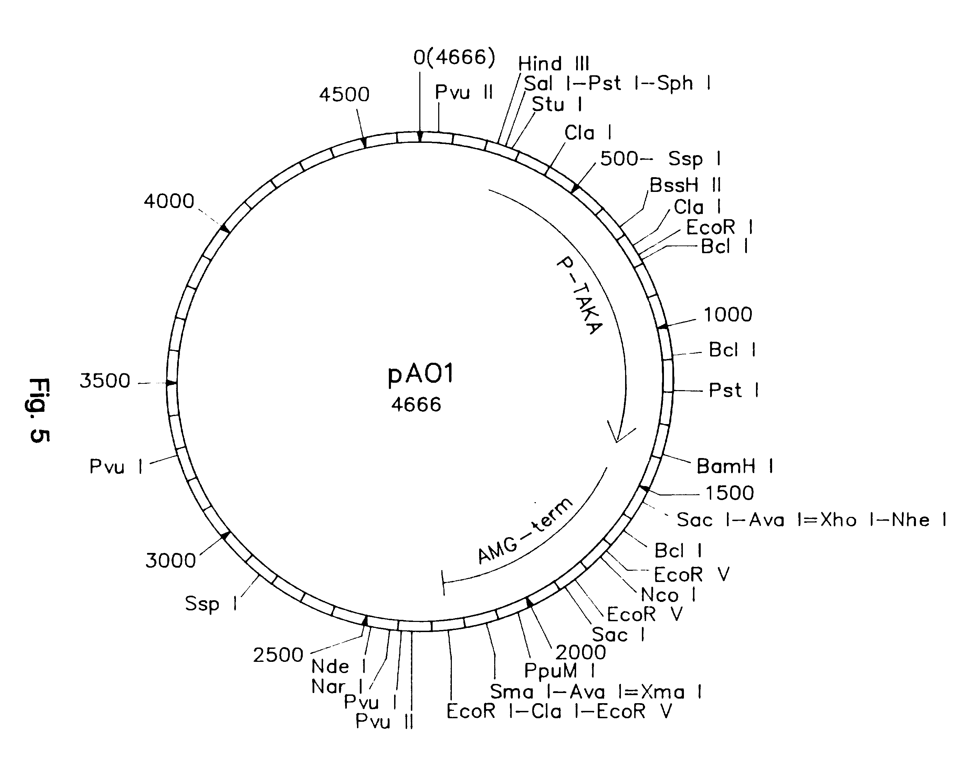

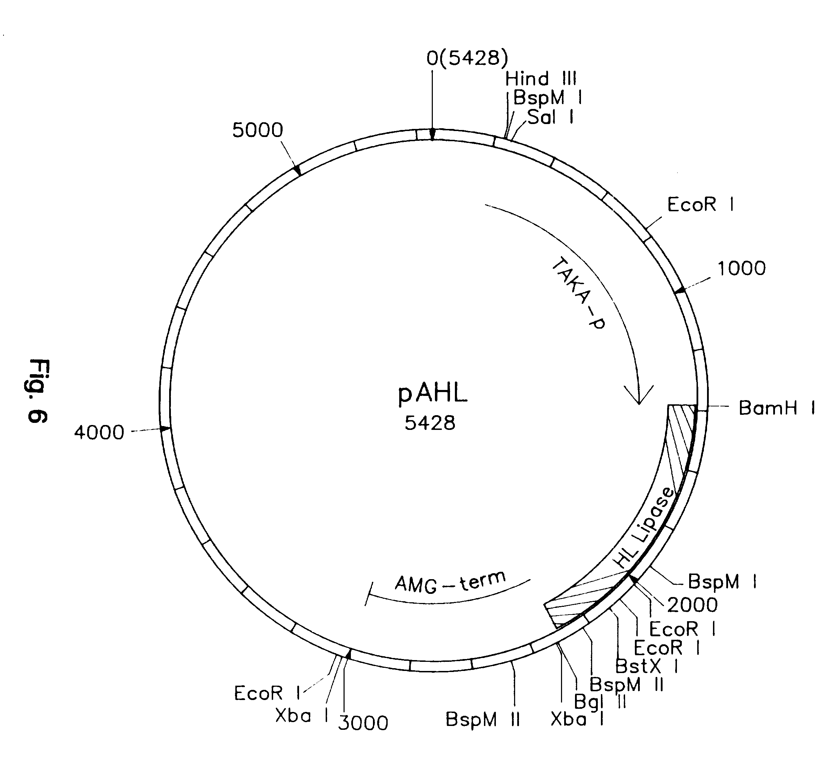

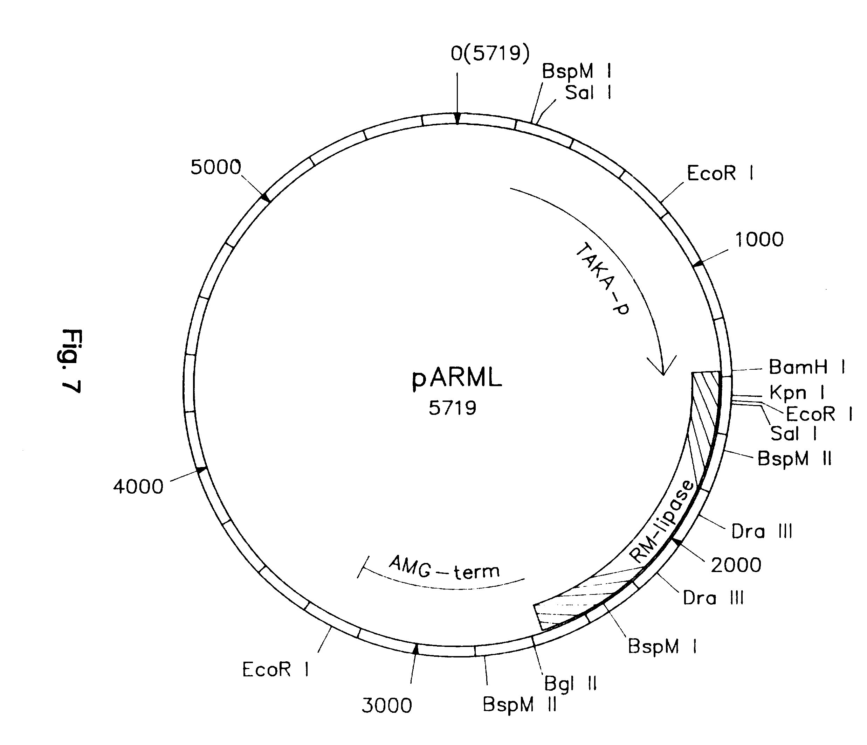

The expression plasmids used in this application are identical to p787 and p960, except for minor modifications immediately 3' to the lipase coding regions. The modifications were made in the following way: p960 was digested with NruI and BamHI restriction enzymes. Between these two sites the BamHI/NheI fragment from plasmid pBR322, in which the NheI fragment was filled in with Klenow polymerase, was cloned, thereby creating plasmid pAOI (Fig. 5) which contains unique BamHI and NheI sites. Between these unique sites BamHI/XbaI fragments from p960 and p787 were cloned to give pAHL (Fig. 6) and pARML (Fig. 7), respectively.

**Site-directed in vitro mutagenesis of lipase genes:**

Three different approaches were used for introducing mutations into the lipase genes.

One method employed was oligonucleotide site-directed mutagenesis which is described by Zoller & Smith, DNA, Vol. 3, No. 6, 479-488 (1984). The method is briefly described in the following, and is described thoroughly in example 1.

Isolated from the expression plasmid, the lipase gene of interest is inserted into a circular M13 bacteriophage vector. To the single-stranded genome, a chemically synthesized complementary DNA-strand is annealed. This DNA-strand contains the mutation to be introduced flanked by sequences complementary to lipase sequences on the circular DNA. In vitro, the primer is then extended in the entire length of the circular genome biochemically using Klenow polymerase. When transformed in E.coli, the heteroduplex will give rise to double-stranded DNA with the desired sequence from which a fragment can be isolated and re-inserted into the expression plasmid.

Another method employed is described in Nelson & Long, Analytical Biochemistry, 180, 147-151 (1989). It involves the 3-step generation of a PCR (polymerase chain reaction) fragment containing the desired mutation introduced by using a chemically synthesized DNA-strand as one of the primers in the PCR-reactions. From the PCR-generated fragment, a DNA fragment carrying the mutation can be isolated by cleavage with restriction enzymes and re-inserted into the expression plasmid. This method is thoroughly described in example 3. In figures 3 and 4 the method is further outlined.

In a further method, usually termed "cassette mutagenesis", a segment between two restriction sites of the lipase-encoding region is replaced by a synthetic DNA fragment carrying the desired mutation.

**Example 1: Construction of a plasmid expressing the D96L variant of Humicola lanuginosa lipase.**

**Isolation of the lipase gene:**

The expression plasmid p960 contains the coding region for Humicola lanuginosa lipase on a BamHI/XbaI restriction fragment (the DNA and amino acid sequence of the lipase are shown in the appended Sequence Listing ID No. 1). The BamHI/XbaI fragment was isolated as follows: The expression plasmid was incubated with the restriction endonucleases BamHI and XbaI. The conditions were: 5 $\mu$g plasmid, 10 units of BamHI, 10 units of XbaI, 100 mM NaCl, 50 mM Tris-HCl, pH 7.5, 10 mM $MgCl_2$ and 1 mM DTT in 50 $\mu$l volume. The temperature was 37°C and the reaction time 2 hours. The two fragments were separated on a 1% agarose gel and the desired fragment was isolated from the gel.

**Ligation to the vector M13mp18:**

The bacteriophage vector M13mp18 on its double-stranded, replicative form was digested with BamHI and XbaI under conditions as described above. The isolated restriction fragment was ligated to the digested bacteriophage vector in the following reaction mixture: Fragment 0.2 $\mu$g, vector 0.02 $\mu$g, 50 mM Tris-HCl, pH 7.4, 10 mM $MgCl_2$, 10 mM DTT and 1 mM ATP in a 20 $\mu$l volume at 16°C for 3 hours. 5 $\mu$l of this mixture was transformed into the E.coli strain JM101. The presence of fragment in the vector was identified by restriction enzyme analysis on double-stranded M13-DNA isolated from the transformants.

**Isolation of single-stranded (ss) DNA (template):**

From the transformant described above, ss-DNA was isolated according to a method described by Messing in Gene, 19, 269-276 (1982).

**5' phosphorylation of the mutagenisation primer:**

The mutagenisation primer with the sequence 5'-TTTCTTTCAACAAGAAGTTAAGA-3' was phosphorylated at the 5' end in a 30 μl reaction mixture containing 70 mM Tris-HCl, pH 7.0, 10 mM $MgCl_2$, 5 mM DTT, 1 mM ATP, 100 pmol oligonucleotide and 3.6 units of T4 polynucleotide kinase. The reaction was carried out for 30 min. at 37°C. Then, the enzyme was inactivated by incubating the mixture for 10 min. at 65°C.

**Annealing of template and phosphorylated mutagenisation primer:**

Annealing of template and primer was carried out in a 10 μl volume containing 0.5 pmol template, 5 pmol primer, 20 mM Tris-HCl, pH 7.5, 10 mM $MgCl_2$ 50 mM NaCl and 1 mM DTT by heating for 10 min. at 65°C and cooling afterwards to 0°C.

**Extension/ligation reaction:**

To the reaction mixture above, 10 μl of the following mixture was added: 0.3 mM dATP, 0.3 mM dCTP, 0.3 mM dGTP, 0.3 mM TTP, 1 mM ATP, 20 mM Tris-HCl, pH 7.5, 10 mM $MgCl_2$, 10 mM DTT, 3 units of T4 DNA ligase and 2.5 units of Klenow polymerase. Then, the reaction was carried out for 16 hours at 16°C.

**Transformation of JM101:**

The reaction mixture above was transformed in different dilutions into $CaCl_2$-treated E.coli JM101 cells using standard techniques and plated in 2 x YT top agar on 2 x YT agar plates. (2 x YT = tryptone 16 g/l, yeast extract 10 g/l, NaCl 5 g/l. 2 x YT topagar = 2 x YT with 0.4% agarose added and autoclaved. 2 x YT agar plates = 2 x YT with 2% agar added and autoclaved). The plates were incubated at 37°C overnight.

**Identification of positive clones:**

The method used was plaque-lift hybridization which is described in the following: a nitrocellulose filter was placed on a plate with a suitable plaque-density, so that the filter was wetted. The filter was then bathed in the following solutions: 1.5 M NaCl, 0.5 M NaOH for 30 sec., 1.5 M NaCl, 0.5 M Tris-HCl, pH 8.0 for 1 min. and 2 x SSC (0.3 M NaCl, 0.03 M sodium citrate) till later use. The filter was dried on 3MM filter paper and baked for 2 hours at 80°C in a vacuum oven.

The mutagenisation primer with the sequence 5'-TTTCTTTCAACAAGAAGTTAAGA-3' was labelled radioactively at the 5'-end in a 30 μl volume containing 70 mM Tris-HCl, pH 7.5, 10 mM $MgCl_2$, 5 mM DTT, 10 pmol oligonucleotide, 20 pmol γ-32P-ATP and 3.5 units of T4 polynucleotide kinase. The mixture was incubated at 37°C for 30 min. and then for 5 min. at 100°C.

The dried filter was prehybridised for 2 hours at 65°C in 6 x SSC, 0.2% bovine serum albumin, 0.2% Ficoll, 0.2% polyvinylpyrrolidon, 0.2% sodium-dodecyl-sulphate (SDS) and 50 μg/ml sonicated salmon sperm DNA. Then, the reaction mixture containing the labelled probe was added to 15 ml of fresh pre-hybridization mix, and the filter was bathed herein overnight at 27°C with gentle shaking. After hybridisation, the filter was washed 3 times each 15 min. in 2 x SSC, 0.1% SDS and autoradiographed. After wash in the same solution, but now at 50°C, and another autoradiography, plaques containing DNA-sequences complementary to the mutagenisation primer were identified.

Because the identified clone is a result of a heteroduplex, the plaque was plated again. The hybridisation and identification steps were repeated.

**Purification of double-stranded M13-phage DNA:**

A re-screened clone was used for infection of E.coli strain JM101. A culture containing approximately $10^8$ phages and 5 colonies of JM101 was grown for 5 hours in 5 ml 2 x YT medium at 37°C. Then, double-stranded, circular DNA was purified from the pellet according to a method described by Birnboim & Doly, Nucleic Acids Res., 2, 1513 (1979).

**Isolation of a restriction fragment encoding modified lipase:**

The DNA preparation (appr. 5 μg) isolated above was digested with 10 units of each of the restriction endonucleases BamHI and XbaI in 60 μl of 100 mM NaCl, 50 mM Tris-HCl, pH 7.5, 10 mM $MgCl_2$ and 10 mM DTT for 2 hours at 37°C. The DNA products were separated on an agarose gel end the fragment was purified from the gel.

**Ligation to the Aspergillus expression vector pAO1 (figure 5):**

The isolated restriction fragment was ligated to the Aspergillus vector pAO1 digested with the restriction enzymes BamHI and NheI in the following reaction mixture: Fragment 0.2 μg, vector 0.02 μg, 50 mM Tris-HCl, pH 7.4, 10 mM $MgCl_2$, 10 mM DTT, 1 mM ATP in a total volume of 20 μl. 5 μl of this reaction mix was used for transformation of E.coli strain MC1061, in which the modified expression plasmid was identified and propagated. The plasmid was called pAHLD96L and is identical to pAHL except for the modified codon.

**Sequence verification of pAHLD96L:**

The mutagenized plasmid was sequenced directly on the double-- stranded plasmid using the dideoxy chain termination method originally described by Sanger.

**Example 2: Construction of plasmids expressing other variants of Humicola lipase.**

Other mutant lipase genes were constructed using the same method as described in example 1. Plasmid names and primers used for the modifications are listed below.

| Plasmid name | Primer sequence |
|---|---|
| pAHLD96N | 5′-TCTTTCAAGTTGAAGTTAAGA-3′ |
| pAHLD111N | 5′-GTGAAGCCGTTATGTCCCCTG-3′ |
| pAHLE87Q | 5′-CGATCCAGTTTTGTATGGAACGA-3′ |
| pAHLR209A/E210A | 5′-GCTGTAACCGAAAGCAGCCGGCGGGAGTCT-3′ |
| pAHLE87A | 5′-CGATCCAGTTAGCTATGGAACG-3′ |
| pAHLE56A | 5′-CTCCAGAGTCAGCAAACGAGTA-3′ |
| pAHLE56Q | 5′-CCAGAGTCTTGAAACGAGTAG-3′ |
| pAHLD111L | 5′-AAGTGAAGCCCAAATGTCCCCTG-3′ |
| pAHLE210A | 5′-TGTAACCGAAAGCGCGCGGCGG-3′ |
| pAHLE210Q | 5′-TAACCGAATTGGCGCGGCGGG-3′ |
| pAHLR209A | 5′-AACCGAATTCAGCCGGCGGGAGT-3′ |

**Example 3: Construction of a plasmid expressing the D254N variant of Humicola lanuginosa lipase.**

**Linearization of plasmid pAHL:**

The circular plasmid pAHL was linearized with the restriction enzyme SphI in the following 50 μl reaction mixture: 50 mM NaCl, 10 mM Tris-HCl, pH 7.9, 10 mM $MgCl_2$, 1 mM dithiothreitol, 1 μg plasmid and 2 units of SphI. The digestion was carried out for 2 hours at 37°C. The reaction mixture was extracted with phenol (equilibrated with Tris-HCl, pH 7.5) and precipitated by adding 2 volumes of ice-cold 96% ethanol. After centrifugation and drying of the pellet, the linearized DNA was dissolved in 50 μl $H_2O$ and the concentration estimated on an agarose gel.

**3-step PCR mutagenesis:**

As shown in Fig. 4, 3-step mutagenisation involves the use of four primers:

```
Mutagenisation primer (=A):    5'-GTGCGCAGGGATGTTCGGAATGTTAGG-3'


PCR Helper 1 (=B):   5'-GGTCATCCAGTCACTGAGACCCTCTACCTATTAAATCGG-
C-3'


PCR Helper 2 (=C):   5'-CCATGGCTTTCACGGTGTCT-3'
PCR Handle (=D):     5'-GGTCATCCAGTCACTGAGAC-3'
```

All 3 steps were carried out in the following buffer containing: 10 mM Tris-HCl, pH 8.3, 50 mM KCl, 1.5 mM MgCl$_2$, 0.001% gelatin, 0.2 mM dATP, 0.2 mM dCTP, 0.2 mM dGTP, 0.2 mM TTP, 2.5 units Taq polymerase.

In step 1, 100 pmol primer A, 100 pmol primer B and 1 fmol linearized plasmid was added to a total of 100 µl reaction mixture and 15 cycles consisting of 2 minutes at 95°C, 2 minutes at 37°C and 3 minutes at 72°C were carried out.

The concentration of the PCR product was estimated on an agarose gel. Then, step 2 was carried out. 0.6 pmol step 1 product and 1 fmol linearized plasmid was contained in a total of 100 µl of the previously mentioned buffer and 1 cycle consisting of 5 minutes at 95°C, 2 minutes at 37°C and 10 minutes at 72°C was carried out.

To the step 2 reaction mixture, 100 pmol primer C and 100 pmol primer D was added (1 µl of each) and 20 cycles consisting of 2 minutes at 95°C, 2 minutes at 37°C and 3 minutes at 72°C were carried out. This manipulation comprised step 3 in the mutagenisation procedure.

**Isolation of mutated restriction fragment:**

The product from step 3 was isolated from an agarose gel and re-dissolved in 20 µl H$_2$O. Then, it was digested with the restriction enzyme BspMII in a total volume of 50 µl with the following composition: 100 mM NaCl, 50 mM Tris-HCl, pH 7.9, 10 mM MgCl$_2$, 1 mM DTT and 10 units of BspMII. Incubation was at 37°C for 2 hours. The 264 bp BspMIII fragment was isolated from an agarose gel.

**Ligation to expression vector pAHL:**

The expression plasmid pAHL was cleaved with BspMII under conditions indicated above and the large fragment was isolated from an agarose gel. To this vector, the mutated fragment isolated above was ligated and the ligation mix was used to transform E.coli. The presence and orientation of the fragment was verified by cleavage of a plasmid preparation from a transformant with restriction enzymes. Sequence analysis was carried out on the double-stranded plasmid using the di-deoxy chain termination procedure developed by Sanger. The plasmid was named pAHLD254N and is identical to pAHL, except for the altered codon.

**Example 4: Construction of plasmids expressing other variants of Humicola lipase.**

The following mutants were constructed using the same method as described in example 3, except other restriction enzymes were used for digesting the PCR-product and the vector used for recloning of the mutated fragment. Plasmid names and primers used for the modifications are listed below.

```
Plasmid name              Primer A sequence
pAHLD254K          5'-GTGCGCAGGGATCTTCGGAATGTT-3'
pAHLD254R          5'-GTGCGCAGGGATTCTCGGAATGTT-3'
pAHLD242N          5'-GCCGCCGGTGGCGTTGATGCCTTCTAT-3'
pAHLD242N/D254N        5'GTGCGCAGGGATGTTCGGAATGTTAGGCTGGTTATTGCCG-
                       CCGGTGGCGTTGATGCCTTCTAT-3'
pAHLE87R           5'-CCCGATCCAGTTTCTTATCGATCGAGAGCCGCGG-3'
pAHLE87K           5'-CGATCCAGTTCTTTATCGATCGAGAGCCACGG-3'
```

## Example 5: Construction of lipase variants by combination of available mutants:

The following mutants were constructed by combining plasmid fragments of mutants constructed above. For example, pAHLE87K/D254K was constructed by isolating the BamHI/BstXI restriction fragment from pAHLE87K and inserting the fragment into pAHLD254K digested with BamHI and BstXI:

```
Plasmid
pAHLE87K/D254K
pAHLE87Q/D254N/D242N/E210Q
pAHLE87Q/D242N/E210Q
pAHLR209A/E210A/D96L
pAHLR209A/E210Q/E56Q
pAHLE210Q/D242N/D254N
pAHLE87Q/E210Q/D242N
```

## Example 6

## Transformation of Aspergillus oryzae (general procedure)

100 ml of YPD (Sherman et al., Methods in Yeast Genetics, Cold Spring Harbor Laboratory, 1981) was inoculated with spores of A. oryzae and incubated with shaking for about 24 hours. The mycelium was harvested by filtration through miracloth and washed with 200 ml of 0.6 M $MgSO_4$. The mycelium was suspended in 15 ml of 1.2 M $MgSO_4$, 10 mM $NaH_2PO_4$, pH = 5.8. The suspension was cooled on ice and 1 ml of buffer containing 120 mg of Novozym® 234, batch 1687 was added. After 5 min., 1 ml of 12 mg/ml BSA (Sigma type H25) was added and incubation with gentle agitation continued for 1.5 - 2.5 hours at 37°C until a large number of protoplasts was visible in a sample inspected under the microscope.

The suspension was filtered through miracloth, the filtrate transferred to a sterile tube and overlayed with 5 ml of 0.6 M sorbitol, 100 mM Tris-HCl, pH = 7.0. Centrifugation was performed for 15 min. at 1000 g and the protoplasts were collected from the top of the $MgSO_4$ cushion. 2 volumes of STC (1.2 M sorbitol, 10 mM Tris-HCl, pH = 7.5, 10 mM $CaCl_2$) were added to the protoplast suspension and the mixture was centrifuged for 5 min. at 1000 g. The protoplast pellet was resuspended in 3 ml of STC and repelleted. This was repeated. Finally, the protoplasts were resuspended in 0.2 - 1 ml of STC.

100 μl of protoplast suspension was mixed with 5 - 25 μg of p3SR2 (an A. nidulans amdS gene carrying plasmid described in Hynes et al., Mol. and Cel. Biol., Vol. 3, No. 8, 1430-1439, Aug. 1983) in 10 μl of STC. The mixture was left at room temperature for 25 min. 0.2 ml of 60% PEG 4000 (BDH 29576), 10 mM $CaCl_2$ and 10 mM Tris-HCl, pH = 7.5 was added and carefully mixed (twice) and finally 0.85 ml of the same solution was added and carefully mixed. The mixture was left at room temperature for 25 min., spun at 2.500 g for 15 min. and the pellet was resuspended in 2 ml of 1.2 M sorbitol. After one more sedimentation the protoplasts were spread on minimal plates (Cove, Biochem. Biophys. Acta 113 (1966) 51-56) containing 1.0 M sucrose, pH = 7.0, 10 mM acetamide as nitrogen source and 20 mM CsCl to inhibit background growth. After incubation for 4 - 7 days at 37°C spores were picked, suspended in sterile water and spread for single colonies. This procedure was repeated and spores of a single colony after the second reisolation were stored as a defined transformant.

## Example 7

### Expression of the lipase variant D96L in A. oryzae

pAHLD96L was transformed into A. oryzae IFO 4177 by cotransformation with p3SR2 containing the amdS gene from A. nidulans as described in example 15. Protoplasts prepared as described were incubated with a mixture of equal amounts of pAHLD96L and p3SR2, approximately 5 μg of each were used. 9 transformants which could use acetamide as sole nitrogen source were reisolated twice. After growth on YPD for three days, culture supernatants were analyzed using the assay for lipase activity described in example 16 (Purification of lipase variants of the invention). The best transformant was selected for further studies and grown in a 1 1 shake-flask on 200 ml FG4 medium (3% soy meal, 3% maltodextrin, 1% peptone, pH adjusted to 7.0 with 4 M NaOH) for 4 days at 30°C. Under these conditions the transformant gave about 500 lipase units per ml of culture.

The other lipase variants were produced essentially as described above, using the general procedure described in example 6.

## Example 8

### Purification of lipase variants of the invention

Assay for lipase activity :

A substrate for lipase was prepared by emulsifying glycerine tributyrat (MERCK) using gum-arabic as emulsifier. Lipase activity was assayed at pH 7 using pH stat method. One unit of lipase activity (LU/mg) was defined as the amount needed to liberate one micromole fatty acid per minute.

Step 1:- Centrifuge the fermentation supernatant, discard the precipitate. Adjust the pH of the supernatant to 7 and add gradually an equal volume of cold 96 % ethanol. Allow the mixture to stand for 30 minutes in an ice bath. Centrifuge and discard the precipitate.

Step 2:- Ion exchange chromatography. Filter the supernatant and apply on DEAE-fast flow (Pharmacia TM) column equilibrated with 50 mM tris-acetate buffer pH 7. Wash the column with the same buffer till absorption at 280 nm is lower than 0.05 OD. Elute the bound enzymatic activity with linear salt gradient in the same buffer (0 to 0.5 M NaCl) using five column volumes. Pool the fractions containing enzymatic activity .

Step 3:- Hydrophobic chromatography. Adjust the molarity of the pool containing enzymatic activity to 0.8 M by adding solid Ammonium acetate. Apply the enzyme on TSK gel Butyl- Toyopearl 650 C column (available from Tosoh Corporation Japan) which was pre-equilibrated with 0.8 M ammonium acetate. Wash the unbound material with 0.8 M ammonium acetate and elute the bound material with distilled water.

Step 4:- Pool containing lipase activity is diluted with water to adjust conductance to 2 mS and pH to 7. Apply the pool on High performance Q Sepharose (Pharmacia) column pre-equilibrated with 50 mM tris -acetate buffer pH 7. Elute the bound enzyme with linear salt gradient.

## Example 9

### The washing performance of lipase variants of the invention

The washing performance of Humicola lanuginosa lipase variants of the invention was evaluated on the basis of the enzyme dosage in mg of protein per litre according to $OD_{280}$ compared to the wild-type H. lanuginosa lipase.

Wash trials were carried out in 150 ml beakers placed in a thermostated water bath. The beakers were stirred with triangular magnetic rods.

The experimental conditions were as follows:

Method:          3 cycles with overnight drying between each cycle
Wash liquor:     100 ml per beaker
Swatches:        6 swatches (3.5 x 3.5 cm) per beaker

| Fabric: | 100% cotton, Test Fabrics style #400 |
| Stain: | Lard coloured with Sudan red (0.75 mg dye/g of lard). 6 µl of lard heated to 70°C was applied to the centre of each swatch. After application of the stain, the swatches were heated in an oven at 75°C for 30 minutes. The swatches were then stored overnight at room temperature prior to the first wash. |
| Detergent: | LAS (Nansa 1169/P, 30% a.m.) 1.17 g/l |
| | AEO (Dobanol 25-7) 0.15 g/l |
| | Sodium triphosphate 1.25 g/l |
| | Sodium sulphate 1.00 g/l |
| | Sodium carbonate 0.45 g/l |
| | Sodium silicate 0.15 g/l |
| pH: | 10.2 |
| Lipase conc.: | 0.075, 0.188, 0.375, 0.75 and 2.5 mg of lipase protein per litre |
| Time: | 20 minutes |
| Temperature: | 30°C |
| Rinse: | 15 minutes in running tap water |
| Drying: | overnight at room temperature (~20°C, 30-50% RH) |
| Evaluation: | after the 3rd wash, the reflectance at 460 nm was measured. |

**Results**

Dose-response curves were compared for the lipase variants and the native <u>H. lanuginosa</u> lipase. The dose-response curves were calculated by fitting the measured data to the following equation:

$$\Delta R = \Delta R_{max} \frac{C^{0.5}}{K + C^{0.5}} \tag{I}$$

where

$\Delta R$ is the effect expressed in reflectance units
C is the enzyme concentration (mg/l)
$\Delta R_{max}$ is a constant expressing the maximum effect
K is a constant; $K^2$ expresses the enzyme concentration at which half of the maximum effect is obtained.

Based on the characteristic constants $\Delta R_{max}$ and K found for each lipase variant as well as the wild-type lipase, improvement factors were calculated. The improvement factor, defined as

$$f_{improve} = C_{WT}/C \tag{II}$$

expresses the amount of lipase variant protein needed to obtain the same effect as that obtained with 0.25 mg/l of the reference wild-type protein ($C_{WT}$).

Thus, the procedure for calculating the improvement factor was as follows:

1) The effect of the wild-type protein at 0.25 mg/l ($\Delta R_{wild\text{-}type}$) was calculated by means of equation (I);
2) the concentration of lipase variant resulting in the same effect as the wild-type at 0.25 mg/l was calculated by means of the following equation:

$$C = (k_{(variant)} \frac{\Delta R_{(wild\text{-}type)}}{\Delta R_{max(variant)} - \Delta R_{(wild\text{-}type)}})^2 \tag{III}$$

3) the improvement factor was calculated by means of equation (II).

The results are shown in Table 1 below.

Table 1

| Variant | Improvement factor |
|---------|-------------------|
| D96L | 4.4 |
| D111L | 1.0 |
| E87A | 1.0 |
| E56A | 1.6 |
| E56Q | 2.6 |
| R209A | 1.1 |
| D242N | 1.7 |
| R209A+E210A | 1.9 |
| R209A+E210A+D96L | 2.8 |
| E210Q+D242N+D254N | 1.8 |
| R209A+E210A+D96L+E56Q | 1.5 |

It appears from Table 1 that the lipase variants R209A+E210A, E56Q and D96L have a considerably better wash performance than the wild-type lipase. This might possibly be ascribed to the decreased negative charge and increased hydrophobicity of these variants resulting in increased adsorption during washing and consequently higher activity during the drying phase. The performance of the lipase variants E87A, DIIIL and R209A in on a par with that of the wild-type enzyme.

Example 10

**Increased Thermostability of Lipase Variants**

The thermostability of selected variants of H. lanuginosa lipase has been examined by Differential Scanning Calorimetry (DSC). Using this technique, the thermal denaturation temperature, **Td**, is determined by heating an enzyme solution at a constant programmed rate.

**Experiments:**

The Differential Scanning Calorimeter, MC-2D, from MicroCal Inc. was used for the investigations. 50 mM buffer solutions in was prepared at the following pH-values: 4 (acetate), 7 (TRIS-acetate), 10 (glycine). The enzyme concentration ranged between 0.6 - and 0.9 mg/ml, and a total volume of ca. 1.2 ml was used for each experiment. All samples were heated from 5'C to 95'C at a scan rate of 90°C/hr..

**Results:**

The results for the wild type and selected mutants are shown in the table below.

| No | Mutation | pH 4 | | pH7 | | pH 10 | |
|----|----------|------|-----|-----|--------|-------|-----|
| | | Td | dTd | Td | $dT_d$ | Td | dTd |
| **WT** | **-** | 58.9 | - | 74.7 | - | 69.3 | - |
| 1 | **F211A** | 60.2 | +1.3 | 75.8 | +1.1 | 70.3 | +1.0 |
| 2 | **T267R** | 59.4 | +0.5 | 75.7 | +1.0 | 70.0 | +0.7 |
| 3 | **D111N** | 58.3 | -0.6 | 75.6 | +0.9 | 69.9 | +0.6 |
| 4 | **F211L** | 57.8 | -1.1 | 74.8 | 0.1 | 69.4 | 0.1 |
| **Note:** dTd denotes the the change in thermostability as a result of the mutation. | | | | | | | |

Example 11

**Storage stability of H. lanuginosa lipase variants in liquid detergent.**

Several variants were tested in a model liquid detergent with the following composition:

|  |  | % w/w |
|---|---|---|
| Anionic | LAS | 10 |
|  | AS | 1 |
|  | Soap | 14 |
| Nonionic | AEO | 13 |
| Solvent | 1,2-propane diol | 3 |
|  | Ethanol | 5 |
| Buffer | TEA | 6 |
| Builder | Sodium citrate | 1 |
| Neutr.agent | NaOH | 2 |
| Stabilizer etc. | SXS | 1 |
|  | $Ca^{2+}$ | 0,0025 |
|  | Phosphonate | 0,4 |
|  | $Na_2SO_4$ | 0,2 |
| Water | add to 100% |  |
| pH |  | 8 or 10 |

1000 LU per gram of detergent was added and in some samples 0.025 AU/g (Alcalase®) was added. Samples were stored according to the following scheme (triplicate of each):

| Storage temperature: | -18°C | 30°C |
|---|---|---|
| Detergent |  |  |
| pH 8 , no protease | 2 & 7 days | 2 & 7 days |
| pH 8 , 0.025 AU/g |  | 2 days |
| pH 10, no protease | 7 days | 7 days |

Following this incubation the samples were analyzed according to the LU-method (Novo Nordisk AF 95.5).

Assuming that the decay of lipase activity follows a first order kinetic, the rate constant of the decay can be determined:

$$A(t) = A_0 * exp(-k * t)$$

$A(t)$ being the enzyme activity at time t, $A_0$ the initial activity and k the first order rate constant.

For the detergent containing protease a rate constant for the proteolysis can be calculated from

$$A(t) = A_0 * exp(-[k+k_p] * t)$$

where $k_p$ is the rate constant of proteolysis, and where k is calculated from the stability data determined in the detergent without protease.

In each experiment the wild-type H. lanuginosa lipase was included as a reference, and comparison of the variants with the wild-type is only done within an experiment in order to reduce the uncertainty of variation between experiments. Below the results are given, and the relative improvement of a variant over the wild-type is given as:

$$IF_x = k_{wt}/k_x$$

where IF means Improvement factor, $k_{wt}$ is the rate constant of decay of the wild-type (at the given conditions) and $k_x$ is the corresponding rate constant of the variant in question in the same experiment.

IF expresses the relative improvement in half-life ($IF_x=2$ indicates that the half-life of variant x is twice as long as that of the wild-type in the same experiment).

Based on an estimation of variations of replicates within an experiment an IF < 0.7 or IF > 1.3 is considered significant. The unit of k is (day)$^{-1}$.

| Variant | Experiment no. | pH 8 no prot. k*) IF*) | | pH 8 +Alcalase $k_p$ IF | | pH 10 no prot. k IF | |
|---|---|---|---|---|---|---|---|
| Wildtype | 3 | 0.02 | | 0.48 | | 0.19 | |
| | 5 | 0.02 | | 0.40 | | 0.16 | |
| | 6 | 0.00 | | 0.34 | | 0.09 | |
| | 7 | 0.01 | | 0.52 | | 0.22 | |
| | 8 a | 0.01 | | 0.50 | | 0.09 | |
| | b | 0.01 | | 0.52 | | 0.07 | |
| D96N | 3 | 0.00 | | 0.21 | **2.3** | 0.15 | **1.3** |
| | 5 | 0.02 | | 0.26 | **1.6** | n.d. | |
| D111N | 3 | 0.00 | | 0.50 | **1.0** | 0.16 | **1.2** |
| | 5 | 0.02 | | 0.31 | **1.3** | 0.13 | **1.2** |
| E56Q | 3 | 0.01 | | 0.22 | **2.2** | 0.14 | **1.4** |
| D96L | 6 | 0.01 | | 0.17 | **2.0** | 0.08 | **1.2** |
| | 7 | 0.00 | | 0.23 | **2.3** | 0.09 | **2.6** |
| R209A/E210A/ D96L | 7 | 0.02 | | 0.36 | **1.4** | 0.10 | **2.3** |
| E210Q/D242N/ D254N | 7 | 0.02 | | 0.49 | **1.0** | n.d. | |

*)k in the detergent at pH 8 is in all cases very low, and due to the short storage time (7 days, approx. 90% residual activity) it is not determined very accurately. Hence the IF is not calculated.

In conclusion a number of the tested variants had improved resistance to proteolytic degradation, and they almost all had improved resistance to alkaline conditions.

Example 12

**Specific activity**

A higher specific activity (amounts of substrate molecules cleaved pr. unit time pr. unit amount) than the wild-type (wt) swas measured for the lipase variants shown below. This means that these lipases have a superior performance of hydrolysing the actual substrate.

The lipases were fermented and purified in the same way. The purified lipases were tested in a standard LU assay (Analytical method, internal NOVO NORDISK number AF 95/6-GB 1991.02.07). The sample was analysed twice, and the mean values are tabulated. The amount of protein was estimated by optical density measurements on a Shimadzu spectrofotometer, using the wave-length 280 nm. The sample was regarded as pure when the proportional value of OD280 divided by OD260 was greater than 1.6, together with a single band SDS-polyacrylamid gel electroforesis.

| Humicola lanuginosa | Specific activity LU/OD280 |
|---|---|
| DIIIN | 4290* |
| E56A | 4890* |
| L206V | 4750 |
| R209*/E210* | 6686 |
| R209A/E210A/D96L | 4818 |

* only tested once

(continued)

| Humicola lanuginosa | Specific activity LU/OD280 |
|---|---|
| wt | 3790 |

## Claims

1. An enzymatically active lipase variant of a parent lipase, which parent lipase comprises a trypsin-like catalytic triad including an active serine located in a predominantly hydrophobic, elongated binding pocket of the lipase molecule, and comprising a surface loop structure which covers the active serine when the lipase is in inactive form, which surface loop structure is shifted to expose the active site residues when the lipase is activated thereby creating a lipid contact zone located within the part of the lipase structure containing the active serine residue being constituted by a surface with increased surface hydrophobicity which interacts with the lipid substrate at or during hydrolysis, wherein the electrostatic charge and/or hydrophobicity of the lipid contact zone has been cnanged by deleting or substituting one or more negatively charged amino acid residues of the lipid contact zone by neutral or positively charged amino acid residue(s), and/or by substituting one or more neutral amino acid residues by positively charged amino acid residue(s), and/or by deleting or substituting one or more hydrophilic amino acid residues by hydrophobic amino acid residue(s), provided that said variant lipase is different from variants of such a parent lipase isolatable from *Pseudomonas putida* ATCC 53552, in which the Gln at position 127 has been substituted for Arg and/or the Phe residue at position 207 has been substituted for Thr, Gly, Lys or Ala.

2. A lipase variant according to claim 1, wherein one or more glutamic acid or aspartic acid residues of said lipid contact zone are substituted by glutamine, asparagine, alanine, leucine, valine, serine, threonine, lysine, or arginine.

3. A lipase variant according to claim 1 or 2, wherein the parent lipase is a microbial lipase.

4. A lipase variant according to claim 3, wherein the parent lipase is a fungal lipase, preferably derived from a strain of *Humicola,* or *Rhizomucor.*

5. A lipase variant according to claim 4, wherein the parent lipase is a *Rhizomucor miehei* lipase.

6. A lipase variant according to claim 5, wherein one or more amino acid residues are substituted as follows:

   D91N,K,R,A,V,L,S,T;
   D256N, K, R,A,V, L, S,T;
   D226N,K,R,A,V,L,S,T;
   D61 N,K,R,A,V,L,S,T;

D113N,K,R,A,V,L,S,T;
E201Q,K,R,A,V,L,S,T; or
D243N,K,R,A,V,L,S,T.

7. A lipase variant according to claim 4, wherein the parent lipase is a *Humicola lanuginosa* lipase.

8. A lipase variant according to claim 7, wherein one or more amino acid residues are substituted as follows:

E87Q,K,R,A,N,T,S,L,V;
D254N,K,R,A,Q,T,S,L,V;
D242N,K,R,A,Q,T,S,L,V;
E210Q,K,R,A,N,T,S,L,V;
E56Q,K,R,A,N,T,S,L,V;
D96N,K,R,A,Q,T,S,L,V;
D111N,K,R,A,Q,T,S,L,V;
D62A,Q,N,T,S,K,R,L,V;
E219A,Q,N,T,S,K,R,L,V;
E234A,Q,N,T,S,K,R,L,V;
E57A,Q,N,T,S,K,R,L,V
E99A,Q,N,T,S,K,R,L,V;
D27A,Q,N,T,S,K,R,L,V;
E239A,Q,N,T,S,K,R,L,V;

T267K, R;
S85K, R;
T226K, R;
N88K, R;
N92K,R;
1255K, R;
1202K, R;
L206K,R;
R209A
L259K,R;
V203K,R; or
L227K,R; in particular a lipase variant comprising the following substitutions:

E87Q + D254N + D242N + E210Q;
E87Q + D254N + E210Q;
D96N + E87Q + D254N;
R209A + E210A;
R209A+R210A+D96L; or
E210Q+D242N+D254N

9. A lipase variant according to claim 3, wherein the parent lipase is a yeast lipase, e.g. derived from a strain of *Candida,* or a bacterial lipase, e.g. derived from a strain of *Pseudomonas.*

10. A DNA construct comprising a DNA sequence encoding a lipase variant according to any of claims 1-9.

11. A recombinant expression vector which carries a DNA construct according to claim 10.

12. A cell which is transformed with a DNA construct according to claim 33 or a vector according to claim 11.

13. A cell according to claim 12 which is a fungal cell, e.g. belonging to the genus *Aspergillus,* such as *A. niger, A. oryzae,* or *A. nidulans;* a yeast cell, e.g. belonging to a strain of *Saccharomyces,* such as *S. cerevisiae,* or a methylotrophic yeast from the genera *Hansenula,* such as *H. polymorpha,* or *Phichia,* such as *P. pastors;* or a bacterial cell, e.g. belonging to a strain of *Bacillus,* such as *B. subtilis,* or *B. lentus.*

14. A cell according to claim 12 which is a plant cell, e.g. belonging to the *Solanaceae,* such as *Solanum tuberosum,* or *Nicotiana_tabacum.*

15. A method of producing a lipase variant according to any of claims 1-9, wherein a cell according to any of claims 12-14 is cultured or grown under conditions conducive to the production of the lipase variant, and the lipase variant is subsequently recovered from the culture or plant.

16. A detergent additive comprising a lipase variant according to any of claims 1-9, optionally in the form of a non-dusting granulate, stabilised liquid or protected enzyme.

17. A detergent additive according to claim 16, which contains 0.02-200 mg of enzyme protein/g of the additive.

18. A detergent additive according to claim 16 or 17 which additionally comprises another enzyme such as a protease, amylase, peroxidase and/or cellulase.

19. A detergent composition comprising a lipase variant according to any of claims 1 9.

20. A detergent composition according to claim 19 which additionally comprises another enzyme such as a protease, amylase, peroxidase and/or cellulase.

**Patentansprüche**

1. Enzymatisch aktive Lipase-Variante einer parentalen Lipase, wobei die parentale Lipase eine trypsinartige katalytische Triade unter Einschluß eines aktiven Serins in einer vorwiegend hydrophoben, länglichen Bindungstasche des Lipase-Moleküls sowie eine Oberflächen-Schleifenstruktur umfaßt, die das aktive Serin bedeckt, wenn die Lipase in der inaktiven Form vorliegt, wobei diese Oberflächen-Schleifenstruktur unter Freilegen der Reste am aktiven Zentrum verschoben wird, wenn die Lipase aktiviert wird, wodurch eine Lipid-Kontaktzone innerhalb des Teils der Lipase-Struktur, die den aktiven Serinrest enthält, geschaffen wird, die durch eine Oberfläche mit erhöhter Oberflächenhydrophobizität gebildet wird, die mit dem Lipidsubstrat bei oder während der Hydrolyse in Wechselwirkung tritt, wobei die elektrostatische Ladung und/oder die Hydrophobizität der Lipid-Kontaktzone durch Deletion oder Substitution von einem oder mehreren negativ geladenen Aminosäureresten der Lipid-Kontaktzone durch neutrale oder positiv geladene Aminosäurereste und/oder durch Substitution von einem oder mehreren neutralen Aminosäureresten durch positiv geladene Aminosäurereste und/oder durch Deletion oder Substitution von einem oder mehreren hydrophilen Aminosäureresten durch hydrophobe Aminosäurereste verändert ist, mit der Maßgabe, daß diese Lipase-Variante sich von Varianten einer derartigen parentalen Lipase, die aus *Pseudomonas putida* ATCC 53552 isolierbar ist, unterscheidet, worin das Gln in Position 127 durch Arg substituiert ist und/oder der Phe-Rest in Position 207 durch Thr, Gly, Lys oder Ala substituiert ist.

2. Lipase-Variante nach Anspruch 1, wobei ein oder mehr Glutaminsäure- oder Asparaginsäurereste der Lipid-Kontaktzone durch Glutamin, Asparagin, Alanin, Leucin, Valin, Serin, Threonin, Lysin oder Arginin substituiert sind.

3. Lipase-Variante nach Anspruch 1 oder 2, wobei es sich bei der parentalen Lipase um eine mikrobielle Lipase handelt.

4. Lipase-Variante nach Anspruch 3, wobei es sich bei der parentalen Lipase um eine Pilz-Lipase handelt, die vorzugsweise von einem Stamm von *Humicola* oder *Rhizomucor* abgeleitet ist.

5. Lipase-Variante nach Anspruch 4, wobei es sich bei der parentalen Lipase um eine *Rhizomucor miehei*-Lipase handelt.

6. Lipase-Variante nach Anspruch 5, wobei ein oder mehr Aminosäurereste folgendermaßen substituiert sind:

   D91N,K,R,A,V,L,S,T;
   D256N,K,R,A,V,L,S,T;
   D226N,K,R,A,V,L,S,T;
   D61N,K,R,A,V,L,S,T;
   D113N,K,R,A,V,L,S,T;
   E201Q,K,R,A,V,L,S,T; oder

D243N,K,R,A,V,L,S,T.

**7.** Lipase-Variante nach Anspruch 4, wobei es sich bei der parentalen Lipase um eine *Humicola lanuginosa-* Lipase handelt.

**8.** Lipase-Variante nach Anspruch 7, wobei ein oder mehr Aminosäurereste folgendermaßen substituiert sind:

E87Q,K,R,A,N,T,S,L,V;
D254N,K,R,A,Q,T,S,L,V;
D242N,K,R,A,Q,T,S,L,V;
E210Q,K,R,A,N,T,S,L,V;
E56Q,K,R,A,N,T,S,L,V;
D96N,K,R,A,Q,T,S,L,V;
D111N,K,R,A,Q,T,S,L,V;
D62A,Q,N,T,S,K,R,L,V;
E219A,Q,N,T,S,K,R,L,V;
E234A,Q,N,T,S,K,R,L,V;
E57A,Q,N,T,S,K,R,L,V;
E99A,Q,N,T,S,K,R,L,V;
D27A,Q,N,T,S,K,R,L,V;oder
E239A,Q,N,T,S,K,R,L,V
T267K,R;
S85K,R;
T226K,R;
N88K,R;
N92K,R;
I255K,R;
I202K,R;
L206K,R;
R209A;
L259K,R;
V203K,R; oder
L227K,R insbesondere eine Lipase-Variante mit folgenden Substitutionen:
E87Q + D254N + D242N + E210Q;
E87Q + D254N + E210Q;
D96N + E87Q + D254N;
R209A + E210A;
R209A + R210A + D96L; oder
E210Q + D242N + D254N.

**9.** Lipase-Variante nach Anspruch 3, wobei es sich bei der parentalen Lipase um eine Hefe-Lipase, die beispielsweise von einem Stamm von *Candida* abgeleitet ist, oder um eine bakterielle Lipase, die beispielsweise von einem Stamm von *Pseudomonas* abgeleitet ist, handelt.

**10.** DNA-Konstrukt, umfassend eine DNA-Sequenz, die für eine Lipase-Variante nach einem der Ansprüche 1 bis 9 kodiert.

**11.** Rekombinanter Expressionsvektor, der ein DNA-Konstrukt nach Anspruch 10 trägt.

**12.** Zelle, die mit einem DNA-Konstrukt nach Anspruch 10 oder mit einem Vektor nach Anspruch 11 transformiert ist.

**13.** Zelle nach Anspruch 12, bei der es sich um eine Pilzzelle, z. B. der Gattung *Aspergillus,* wie *A. niger, A. oryzae* oder *A. nidulans;* eine Hefezelle, die beispielsweise zu einem Stamm von *Saccharomyces,* wie *S. cerevisiae,* gehört, oder um eine methylotrophe Hefe der Gattungen *Hansenula,* wie *H. polymorpha* oder *Pichia,* z.B. *P. pastoris;* oder um eine bakterielle Zelle, die beispielsweise zu einem Stamm von *Bacillus,* wie *B. subtilis* oder *B. lentus* gehört, handelt.

**14.** Zelle nach Anspruch 12, bei der es sich um eine Pflanzenzelle handelt, die beispielsweise zu den *Solanaceae,* wie *Solanum tuberosum* oder *Nicotiana tabacum,* gehört.

15. Verfahren zur Herstellung einer Lipase-Variante nach einem der Ansprüche 1 bis 9, wobei man eine Zelle nach einem der Ansprüche 12 bis 14 unter Bedingungen züchtet oder wachsen läßt, die zur Bildung der Lipase-Variante führen, und anschließend die Lipase-Variante aus der Kultur oder Pflanze gewinnt.

16. Waschmitteladditiv, umfassend eine Lipase-Variante nach einem der Ansprüche 1 bis 9, gegebenenfalls in Form eines staubfreien Granulats, einer stabilisierten Flüssigkeit oder eines geschützten Enzyms.

17. Waschmitteladditiv nach Anspruch 16, das 0,02-200 mg Enzymprotein/g Additiv enthält.

18. Waschmitteladditiv nach Anspruch 16 oder 17, das zusätzlich ein weiteres Enzym, z. B. eine Protease, Amylase, Peroxidase und/oder Cellulase, enthält.

19. Waschmittelzusammensetzung, enthaltend eine Lipase-Variante nach einem der Ansprüche 1 bis 9.

20. Waschmittelzusammensetzung nach Anspruch 19, die zusätzlich ein weiteres Enzym, z. B. eine Protease, Amylase, Peroxidase und/oder Cellulase, enthält.

**Revendications**

1. Variant de lipase, d'une lipase parentale, actif de manière enzymatique, dont la lipase parentale comporte un élément trivalent catalytique analogue à la trypsine comportant une sérine active localisée dans une poche de manière prédominante hydrophobe, de liaison allongée de la molécule de lipase, et comportant une structure de surface en boucle qui couvre la sérine active lorsque la lipase est sous sa forme inactive, laquelle structure de surface en boucle est décalée pour exposer les résidus du site actif lorsque la lipase est activée créant de ce fait une zone de contact lipidique localisée au sein de la partie de la structure de lipase contenant le résidu sérine actif étant constituée par une surface ayant une hydrophobicité de surface accrue qui interagit avec le substrat lipidique au niveau de ou pendant l'hydrolyse, dans lequel la charge électrostatique et/ou l'hydrophobicité de la zone de contact lipidique a été changée en délétant ou en substituant un ou plusieurs résidus d'acide aminé chargés négativement de la zone de contact lipidique par un ou par des résidus d'acide aminé neutres ou chargés positivement, et/ou en substituant un ou plusieurs résidus d'acide aminé neutres par un ou par des résidus d'acide aminé chargés positivement, et/ou en délétant ou en substituant un ou plusieurs résidus d'acide aminé hydrophiles par un ou par des résidus d'acide aminé hydrophobes, pourvu que ledit variant de lipase soit différent des variants d'une telle lipase parentale pouvant être isolée à partir d'ATCC 53552 de *Pseudomonas putida,* dans lesquels la Gln au niveau de la position 127 a été substituée à la place d'une Arg et/ou le résidu Phe au niveau de la position 207 a été substitué à la place de Thr, Gly, Lys ou Ala.

2. Variant de lipase selon la revendication 1, dans lequel un ou plusieurs résidus d'acide glutamique ou d'acide aspartique de ladite zone de contact lipidique sont substitués par une glutamine, une asparagine, une alanine, une leucine, une valine, une sérine, une thréonine, une lysine ou une arginine.

3. Variant de lipase selon les revendications 1 ou 2, dans lequel la lipase parentale est une lipase microbienne.

4. Variant de lipase selon la revendication 3, dans lequel la lipase parentale est une lipase fongique, de préférence dérivée d'une souche d'*Humicola*, ou de *Rhizomucor.*

5. Variant de lipase selon la revendication 4, dans lequel la lipase parentale est une lipase de *Rhizomucor miehei.*

6. Variant de lipase selon la revendication 5, dans lequel un ou plusieurs résidus d'acide aminé sont substitués comme suit :

   D91N,K,R,A,V,L,S,T ;
   D256N,K,R,A,V,L,S,T ;
   D226N,K,R,A,V,L,S,T ;
   D61N,K,R,A,V,L,S,T ;
   D113N,K,R,A,V,L,S,T ;

E201Q,K,R,A,V,L,S,T ; ou
D243N,K,R,A,V,L,S,T.

**7.** Variant de lipase selon la revendication 4, dans lequel la lipase parentale est une lipase *d'Humicola lanuginosa.*

**8.** Variant de lipase selon la revendication 7, dans lequel un ou plusieurs résidus d'acide aminé sont substitués comme suit :

E87Q,K,R,A,N,T,S,L,V ;
D254N,K,R,A,Q,T,S,L,V ;
D242N,K,R,A,Q,T,S,L,V ;
E210Q,K,R,A,N,T,S,L,V ;
E56Q,K,R,A,N,T,S,L,V ;
D96N,K,R,A,Q,T,S,L,V ;
D111N,K,R,A,Q,T,S,L,V ;
D62A,Q,N,T,S,K,R,L,V ;
E219A,Q,N,T,S,K,R,L,V ;
E234A,Q,N,T,S,K,R,L,V ;
E57A,Q,N,T,S,K,R,L,V ;
E99A,Q,N,T,S,K,R,L,V ;
D27A,Q,N,T,S,K,R,L,V ; ou
E239A,Q,N,T,S,K,R,L,V.
T267K,R ;
S85K,R ;
T226K,R ;
N88K,R ;
N92K,R ;
I255K,R ;
I202K,R ;
L206K,R ;
L259K,R ;
V203K,R ; ou
L227K,R ; en particulier un variant de lipase comportant les substitutions suivantes :
E87Q + D254N + D242N + E210Q ;
E87Q + D254N + E210Q ;
D96N + E87Q + D254N ;
R209A + E210A
R209A + R210A + D96L ; ou
E210Q + D242N + D254N.

**9.** Variant de lipase selon la revendication 3, dans lequel la lipase parentale est une lipase de levure, par exemple dérivée à partir d'une souche de *Candida,* ou une lipase bactérienne, par exemple dérivée à partir d'une souche de *Pseudomonas.*

**10.** Construction d'ADN comportant une séquence d'ADN codant pour un variant de lipase selon l'une quelconque des revendications 1 à 9.

**11.** Vecteur d'expression recombinant qui porte une construction d'ADN selon la revendication 10.

**12.** Cellule qui est transformée à l'aide d'une construction d'ADN selon la revendication 10 ou un vecteur selon la revendication 11.

**13.** Cellule selon la revendication 12, qui est une celle fongique, par exemple appartenant au genre *Aspergillus,* tel que *A. niger, A. oryzae, ou A. nidulans ;* une cellule de levure, par exemple appartenant à une souche de *Saccharomyces,* telle que *S. cerevisiae,* ou une levure méthylotrophique provenant du genre *Hansenula,* telle que *H. polymorpha,* ou *Phichia,* telle que *P. pastoris ;* ou une cellule bactérienne, par exemple appartenant à une souche de *Bacillus,* telle que *B. subtilis,* ou *B. lentus.*

**14.** Cellule selon la revendication 12, qui est une cellule végétale, par exemple appartenant à *Solana-ceae,* telle que *Solanum tuberosum,* ou *Nicotiana tabacum.*

**15.** Procédé de production d'un variant de lipase selon l'une quelconque des revendications 1 à 9, dans lequel une cellule selon l'une quelconque des revendications 12 à 14 est cultivée ou mise en croissance dans des conditions conduisant à la production du variant de lipase, et le variant de lipase est de manière subséquente récupéré à partir de la culture ou du végétal.

**16.** Additif de détergent comportant un variant de lipase selon l'une quelconque des revendications 1 à 9, de manière optionnelle sous la forme d'un granulé non-poussiéreux, d'un liquide stabilisé ou d'une enzyme protégée.

**17.** Additif de détergent selon la revendication 16, qui contient 0,02 à 200 mg de protéine enzymatique/g d'additif.

**18.** Additif de détergent selon les revendications 16 ou 17, qui comporte de manière supplémentaire une autre enzyme telle qu'une protéase, une amylase, une peroxydase et/ou une cellulase.

**19.** Composition de détergent comportant un variant de lipase selon l'une quelconque des revendications 1 à 9.

**20.** Composition de détergent selon la revendication 19, qui comporte de manière supplémentaire une autre enzyme telle qu'une protéase, une amylase, une peroxydase et/ou une cellulase.

Fig. 1a

Fig. 1b

Fig. 2a

Fig. 2b

Linearize plasmid with unique restriction enzyme

3-step PCR incorporating mutation

EcoRI    SalI

Cleave with restriction enzymes, isolate fragment

Reclone into expression plasmid

Sequence verification of PCR fragment

ACGT

Fig. 3

Fig. 4

Fig. 5

Fig. 6

Fig. 7